# EUROPÄISCHE PATENTSCHRIFT

(11) **EP 0 613 685 B1**
(45) Veröffentlichungstag und Bekanntmachung des Hinweises auf die Patenterteilung: **11.11.1998**
(21) Anmeldenummer: 94102340.0
(22) Anmeldetag: 16.02.1994
(51) Int. Cl.: A61K 9/127, A61K 31/155, A61K 7/00, A61K 7/22

(54) **Liposomen, enthaltend Chlorhexidindiacetat oder Chlorhexidingluconat**
Chlorhexidine diacetate or chlorhexidine digluconate containing liposomes
Liposomes contenant le diacetate ou le digluconate de chlorhexidine

(30) Priorität: 02.03.1993 DE 4306475
(43) Veröffentlichungstag der Anmeldung: 07.09.1994
(73) Patentinhaber: Gonzalez Ensenat, Pedro, E-08021 Barcelona (ES); Maierhofer, Günther, D-81247 München (DE); Olivé Moncho, Jorge, 08017 Barcelona (ES); Echeverria Garcia, José Javier, E-08006 Barcelona (ES)
(72) Erfinder: Gonzalez Ensenat, Pedro, E-08021 Barcelona (ES); Maierhofer, Günther, D-81247 München (DE); Olivé Moncho, Jorge, 08017 Barcelona (ES); Echeverria Garcia, José Javier, E-08006 Barcelona (ES)
(74) Vertreter: Grünecker, Kinkeldey, Stockmair & Schwanhäusser Anwaltssozietät

(56) Entgegenhaltungen:
- EP-A- 0 475 160
- EP-A- 0 509 338
- WO-A-92/20319
- DATABASE WPI Week 8827, Derwent Publications Ltd., London, GB; AN 88-188027 & JP-A-63 126 820 (SHISEIDO KK) 30. Mai 1988

## Beschreibung

Die Erfindung betrifft Liposomen, die zur speziellen Anwendung von Chlorhexidin für Desinfektion und medizinische Zwecke dienen.

Die Häufung bakterieller Plaques auf der Zahnoberfläche oder im Zahnfleischgewebe ist der hauptsächlichehe ätiologische Faktor für beginnenden Zahnverfall, Zahnfleischentzündung und Wurzelhautentzündung. In all diesen Fällen besteht die Vorsorge in erster Linie in der täglichen häuslichen Mundhygiene, d. h. Zähneputzen und Reinigung der Zahnzwischenräume, sowie in regelmäßigen zahnärztlichen Behandlungen, bei denen bakterielle Plaques und Zahnstein meist mit Hilfe von Schleifmaterialien entfernt werden.
Obwohl diese Therapie- und Vorsorgemaßnahmen bei korrekter Durchführung durchaus wirkungsvoll sein können, sind sie doch in mancher Hinsicht beschränkt. So muß die häusliche Mundhygiene regelmäßig und sorgfältig erfolgen. Die Entfernung von Plaques aus den Zahnzwischenräumen erfordert manuelle Geschicklichkeit, die beispielsweise Kinder, ältere Menschen oder Behinderte nicht besitzen. Wie regelmäßig eine Zahnreinigung erfolgt, hängt außerdem stark von Motivation und Erziehung ab. Von noch größerer Bedeutung ist es jedoch, daß die vollständige Entfernung von bakteriellen Plaques und Zahnstein in fortgeschrittenen Fällen von Wurzelgabelung und Zahnfleischtaschen (mit einer Tiefe von mehr als 4 bis 5 mm vom Zahnfleischrand bis zum Grund des Defekts) extreme Schwierigkeiten bereitet. Dies ist selbst dann der Fall, wenn man eine Eingriff durchführt, um einen Zugang für die Reinigung zu erhalten. In vielen Fällen versagt die Behandlung von Periodontitis daher, weil es nicht gelingt, Zahnstein und verunreinigten Wurzelzement aus dem betroffenen Bereich während der Behandlung vollständig zu entfernen, oder weil der Patient nicht in der Lage ist, die Anzahl der bakteriellen Plaques während der Pflegephase unterhalb einer kritischen Grenze zu halten.
Auch die bisher wirkungsvollste chemische Methode, um die Bildung bakterieller Plaques zu verhindern, nämlich die Verwendung von Chlorhexidin als 1,2 bis 2 %ige Lösung zur zweimaligen täglichen Mundspülung von jeweils 2 Minuten, weist Nachteile auf.
Besonders bei Rauchern, Tee- oder Kaffeetrinkern führt diese Methode zu Zahnverfärbungen, die einer täglichen Anwendung entgegenstehen. Zu den weiteren Ursachen der Zahnverfärbung gehören außerdem die Nekrose der Zahnpulpa, endodontische Zahnbehandlungen und die Alterung der Zähne, wobei es in letzterem Fall auch eine Rolle spielt, ob Abbaupartikel von bakteriellen Plaques (gefärbt beispielsweise durch Rauch, Nahrungsmittel oder Getränke) in die äußere Schicht des Zahnschmelzes eingebaut werden. Die relativ weit verbreitete Zahnverfärbung verursacht durch Chlorhexidin ist dosisabhängig. Auch wenn bei der Anwendung einer 0,21 %igen Chlorhexidinlösung, verglichen mit einer 2 %igen, keine so drastische Verfärbung der Zähne auftritt, so bleibt sie doch ein großes, bisher ungelüstes Problem.
Andere Nebenwirkungen von Chlorhexidin, angewandt in der Mundhöhle, sind Geschmacksbeeinträchtigung, exfoliative Irritation der Mundschleimhaut (eine Folge der Alkoholkomponente in der Chlorhexidinlösung, meist 10 % Alkohol) und eine erleichterte Zahnsteinbildung.
Diese Nebenwirkungen schränken den Einsatz von Chlorhexidin stark ein und haben die Suche nach Alternativen stimuliert. Bis heute ist es nicht gelungen, einen anderen Wirkstoff zu finden, der dasselbe Wirkungsspektrum auf die Plaqueinhibierung und die Beseitigung von Plaque hat. Desgleichen ist bisher vergeblich versucht worden, Chlorhexidin haltige Formulierungen so zu verändern, daß keine Nebenwirkungen auftreten.
Die Lösung des Problems wurde erfindungsgemäß dadurch erreicht, daß Chlorhexidin in Liposomen entsprechend der Zusammensetzung in Anspruch 1 verkapselt werden. Spezielle Ausführungen werden durch die Unteransprüche wiedergegeben.

Unter Liposomen werden hier kugelförmige Vesikel aus einer oder mehreren Lipiddoppelschichten mit einem wässrigen Innenraum verstanden. Überraschend wurde festgestellt, daß sich Chlorhexidin in Liposomen, die neben den Doppelschicht bildenden Lipiden zusätzliche Tenside enthalten, stabil verkapseln läßt. Derartig llposomal verkapseltes Chlorhexidin kann nicht nur mit höherer Effizienz spndern auch mit lang andauernder Wirkung eingesetzt werden.
Zur Herstellung der Liposomen sind Doppelschicht bildende Membrankomponenten erforderlich, also amphiphile Substanzen wie Lipide oder Lipoide, d. h. Substanzen mit polaren, hydrophilen Kopfgruppen und unpolaren lipophilen Resten, wie sie für die Liposomenbildung üblicherweise verwendet werden. Zweckmäßig werden vorzugsweise physiologisch verträgliche, natürliche Lipide, wie Steroide, Phospholipide, Sphingolipide und Glykolipide verwendet. Bevorzugte Verbindungen umfassen Cerebroside und Ceramide, natürliche Phosphocholine, Phosphatidsäuren) oder Phosphatidylglycerole, sowie gegebenenfalls Lysophospholipide und Fettsäuren und deren Derivate.

Erfindungsgemäß wurde festgestellt, daß die Chlorhexidin-Liposomen besonders rasch in die Haut und Schleimhaut penetrieren und besonders gut auf Zellen in vivo wirken, wenn den Lipiden, welche die Liposomenmembran bilden, zusätzlich Tenside zugesetzt werden. Die Art der zuzusetzenden Tenside hängt von ihrer physiologischen Verträglichkeit ab. Vorzugsweise werden Substanzen biologischer Herkunft eingesetzt, z. B. Gallensäuren und ihre Derivate, Glukoside, Maltoside und Thioglukoside sowie Lysophospholipide. Besonders bevorzugte Verbindungen sind Natriumcholat, Natriumdesoxycholat, Natriumglykocholat, Natriumtaurocholat, aber auch Polyoxyetylene, Brij 56 und Brij 76, sowie Tween 80. Lipid (L) und Tensid (D) werden zweckmäßig in einem Molverhältnis L/D von 0,5 bis 40 eingesetzt, vorzugsweise 2 bis 20, besonders bevozugt 3,1 bis 5,5.
Erfindungsgemäß werden die Liposomen hergestellt, indem man die lipiden Membrankomponenten und Chlorhexidin entweder als solche oder gelöst in einer geringen Menge eines physiologisch verträglichen, mit Wasser mischbaren Lösungsmittels, z. B. Alkohol, mit einer wässrigen Lösung der Tenside kombiniert. Die wässrige Lösung kann außerdem beispielsweise Salze, wasserlösliche Wirkstoffe oder Puffersubstanzen enthalten. Gegebenenfalls können weitere für die Liposomenherstellung übliche Zusatz- und Hilfsstoffe wie Gelbildner, Membranstabilisatoren und Konservierungsstoffe, z. B. Antioxydantien, sowie Oligopeptide und Proteine eingesetzt werden, die dem System zu jedem beliebigen Zeitpunkt entweder zusammen mit Lipiden und Tensiden oder aber getrennt von ihnen zugeführt werden können.
Die Vesikelbildung erfolgt durch Störung des Systems, indem man der heterogenen Mischung aus Lipiden und Tensiden mechanische Energie zuführt. Dies kann z. B. durch Schütteln, Rühren oder durch andersartige Einwirkung von Scherkräften, z. B. durch Filtrieren, erfolgen. Bevorzugt wird eine Störung des Systems mit Hilfe einmaliger Filtration. Man arbeitet hierbei vorzugsweise bei einem geringen Überdruck von 1 bis 6 bar. Der Porendurchmesser der Filter liegt zweckmäßig zwischen 0,1 und 0,8 µm, vorzugsweise zwischen 0,15 und 0,3 µm. Soll die Liposomenpräparation steril erhalten werden, so beträgt die obere Grenze des Porendurchmessers 0,22 µm. Die Herstellungstemperatur wird der Nutz- und Trägerstoffwahl angepaßt und liegt zweckmäßig zwischen 0 und 95 °C. Vorzugsweise arbeitet man in einem Temperaturbereich von 18 bis 70 °C; besonders bevorzugt für die Lipide mit fluiden Ketten ist der Temperaturbereich zwischen 18 und 38 °C, für die Lipide mit geordneten Ketten zwischen 45 und 60 °C.
Der pH-Wert liegt zweckmäßig in einem Bereich von 1 bis 10. Vorzugsweise wird im pH-Bereich zwischen 4 und 8, besonders bevorzugt zwischen 5 und 6,5 gearbeitet.
Als Ergebnis einer derartigen Präparationsmethode erhält man überwiegend unilamellare Liposomen.
Der Anteil des zu verkapselnden Chlorhexidins ist abhängig vom jeweiligen Anwendungsgebiet und der Löslichkeit des Chlorhexidins. Zweckmäßig liegt die Chlorhexidinkonzentration zwischen 0,01 und 3 %, die der Membranbildner zwischen 0,5 und 10 %.

Die Verkapselung von Chlorhexidin in Liposomen führt zu einer signifikanten Dosisreduktion bei gleicher Wirksamkeit, verglichen mit der freien Form. Nebenwirkungen, wie z. B. die Zahnverfärbung, werden stark reduziert oder treten erst gar nicht auf.
Neben der Beseitigung und zur Vorbeugung von bakterieller Plaquebildung können die erfindungsgemäßen Chlorhexidin-Liposomen auch bei der chemischen Wundbehandlung von Zahnfleischtaschen, bei der Behandlung periodisch auftretender aphtöser Mundschleimhautentzündung, zur periapikalen bakteriellen Dekontamination von Wurzelkanälen bei der endodontischen Therapie, bei Candida-Mykosen an Mund- und Zungenschleimhaut, bei den verschiedenen Formen der Gingivitis und der gingivalen Hyperplasie und bei der bakteriellen Desinfektion epidermaler, mesodermaler und mukosaler Gewebe verwendet werden. Die Anwendung von liposomalem Chlorhexidin ist jedoch nicht auf den Bereich der Zahnmedizin oder der Mundhygiene beschränkt, sondern erstreckt sich auch auf andere medizinische Gebiete, wie z. B. der Wunddesinfektion, präoperative Hautdesinfektion, Augen-, Blasen-, Pleural- und Peritoneal-Spülungen und das Imprägnieren von Gazetupfern.

Mit der erfindungsgemäßen Liposomenpräparation wird demnach ein Mittel zur Verfügung gestellt, das nicht nur eine stabile sondern auch eine wirkungsvollere Anwendungsform von Chlorhexidin darstellt. Weitere Vorteile sind, daß das Verfahren einfach anzuwenden ist und die Chlorhexidinliposomen ausgezeichnet gewebeverträglich sind.

### Beispiel

1 g Chlorhexidindiacetat bzw. Chlorhexidindigluconat und 4 g Sojalecithin werden in 4 ml Ethanol gelöst. 0,04 g Kochsalz und 0,56 g Natriumcholat werden in 43 ml Wasser bidest. gelöst.
Beide Lösungen werden innig vermischt und die heterogene Mischung bei 5 bar sterilfiltriert. Die resultierende Liposomendispersion wird auf den gewünschten pH-Wert eingestellt und auf die in der nachfolgend geschilderten vorläufigen klinischen Cross over-Studie eingesetzte Chlorhexidinkonzentration verdünnnt.
In der Studie wurde liposomales Chlorhexidin mit einer Chlorhexidinkonzentration von 0,05 % (LC) gegen ein handelsübliches Chlorhexidinprodukt mit einer Chlorhexidinkonzentration von 0,12 % (CC) getestet. Eine dritte Gruppe in der Studie wurde mit NaF-Lösung als Placebo behandelt (F).
An dieser Studie nahmen 7 Frauen teil, 21 bis 39 Jahre alt, jede mit mindestens 22 Zähnen, medizinisch gesund und ohne Zahnverfall, Gingivitis oder Periodontitis. Die Studie wurde eingeteilt in 3 Abschnitte von jeweils 4 Tagen. Für jeden Zeitabschnitt wurde folgendes Protokoll durchgeführt:
Nach sorgfältiger, professioneller Plaque-Entfernung, also Beginn mit einem Plaqueindex von Null (gemäß Silness Löe Klassifizierung), wurden die Teilnehmer gebeten, mit dein Zähneputzen aufzuhören und statt dessen Mundspülungen, 2 mal täglich 2 Minuten mit 10 ml einer der drei Lösungen durchzuführen und das über einen Zeitraum von 4 Tagern. Am Ende dieses Zeitabschnitts wurde ein Plaquedetektor (Trace, Lorvic) benutzt und ein bakterieller Plaqueindex für die 12 Vorderzähne bei jedem Teilnehmer bestimmt. Danach wurde den Teilnehmern gestattet, ihr gewohntes Zähneputzen für die folgenden 2 1/2 Tage wieder aufzunehmen, bevor eine neue 4 Tage-Testperiode mit einer der anderen Lösungen begonnen wurde.
Die erhaltenen Daten bezüglich der Plaqueindices wurden an der Abteilung für Statistik der Universität Barcelona ausgewertet, wobei der Kruskel-Wallis Test für die Varianz herangezogen wurde. Die Ergebnisse zeigen einen signifikanten Unterschied zwischen CC und F, ebenso zwischen LC und F (in beiden Fällen Signifikanzlevel = 0,00001). Der Vergleich zwischen CC und LC zeigte keine Unterschiede (Signifikanzlevel = 0,0995). Statistisch signifikante Unterschiede wurden zwischen den Chlorhexidinlösungen und dem Placebo gefunden, wenn alle 3 Testlösungen zusammen ausgewertet wurden (Signifikanzlevel = 0,00001).

Während der Dauer der Studie wurden mit den Chlorhexidin-Liposomen keine Nebenwirkungen beobachtet, wie sie im Allgemeinen mit handelsüblichem Chlorhexidin bemerkt werden.
Basierend auf dem gegenwärtigen Wissensstand über Liposomen als Arzneistoffträger erscheint es vernünftig, sowohl eine substantielle Verbesserung als auch eine stark verbesserte Penetrationsfähigkeit von liposomalem Chlorhexidin zu erwarten, verglichen mit der freien Form des Chlorhexidins.

## Patentansprüche

1. Liposomen mit darin verkapseltem Chlorhexidindiacetat oder Chlorhexidindigluconat, umfassend Doppelschicht-bildende Lipide und Tenside, dadurch gekennzeichnet, daß die Tenside ausgewählt sind aus der Gruppe der Gallensäuren und ihrer Derivate, sowie deren physiologisch verträglichen Salzen.

2. Liposomen nach Anspruch 1, dadurch gekennzeichnet, daß die Lipide und/oder Tenside physiologisch verträgliche Verbindungen, bevorzugt natürlichen Ursprungs, sind.

3. Liposomen nach einem der Ansprüche 1 bis 2, dadurch gekennzeichnet, daß die Lipide ausgewählt sind aus der Gruppe der Phospholipide, Sphingolipide und Glykolipide.

4. Liposomen nach mindestens einem der Ansprüche 1 bis 3, dadurch gekennzeichnet, daß die Tenside ausgewählt sind aus der Natriumcholat, Natriumdeoxycholat, Natriumglykocholat, Natriumtaurocholat oder Natriumtaurodeoxycholat umfassenden Gruppe.

5. Liposomen nach mindestens einem der Ansprüche 1 bis 4, dadurch gekennzeichnet, daß das molare Verhältnis von Lipiden (L) zu Tensiden (D) 0,5 bis 40, vorzugsweise 2 bis 20, besonders bevorzugt 3,1 bis 5,5, beträgt.

6. Liposomen nach mindestens einem der Ansprüche 1 bis 5, dadurch gekennzeichnet, daß sie zusätzlich mindestens einen der üblichen Hilfs- und Zusatzstoffe, vorzugsweise Gelbildner und Membranstabilisatoren, umfassen.

7. Liposomen nach mindestens einem der Ansprüche 1 bis 6, dadurch gekennzeichnet, daß die Doppelschicht-bildenden Lipide und Chlorhexidin entweder als solche oder gelöst in einer geringen Menge eines mit Wasser mischbaren und zweckmäSig physiologisch verträglichen Lösungsmittels mit einer wässrigen Lösung von Tensiden kombiniert werden und die Liposomenbildung in der erhaltenen heterogenen Mischung durch Zufuhr mechanischer Energie, insbesondere durch Schütteln, Rühren oder Filtrieren, eingeleitet wird.

8. Verwendung Von Liposomen nach einem der Ansprüche 1 bis 7 zum Herstellen eines Medikamentes zur Prophylaxe und/oder Behandlung von bakterieller Plaquebildung, zur chemischen Wundbehandlung von Zahnfleischtaschen, zur Behandlung periodisch auftretender aphtöser Mundschleimhautentzündungen, zur periapikalen bakteriellen Dekontamination von Wurzelkanälen bei der endodontischen Therapie, zur Behandlung von Candida-Mykosen an Mund- und Zungenschleimhaut, zur Behandlung der Gingivitis und der gingivalen Hyperplasie und zur bakteriellen Desinfektion epidermaler, mesodermaler und mukosaler Gewebe.

## Claims

1. Liposomes containing chlorhexidine diacetate or chlorhexidine digluconate encapsulated therein, comprising lipids which form a double layer and surfactants, characterised in that the surfactants are selected from among the bile acids and their derivatives, and from the physiologically well-tolerated salts thereof.

2. Liposomes according to claim 1, characterised in that the lipids and/or surfactants are physiologically well-tolerated compounds, preferably of natural origin.

3. Liposomes according to one of claims 1 to 2, characterised in that the lipids are selected from among the phospholipids, sphingolipids and glycolipids.

4. Liposomes according to at least one of claims 1 to 3, characterised in that the surfactants are selected from the group comprising sodium cholate, sodium deoxycholate, sodium glycocholate, sodium taurocholate or sodium taurodeoxycholate.

5. Liposomes according to at least one of claims 1 to 4, characterised in that the molar ratio of lipids (L) to surfactants (D) is from 0.5 to 40, preferably from 2 to 20 and particularly preferably from 3.1 to 5.5.

6. Liposomes according to at least one of claims 1 to 5, characterised in that they contain in addition at least one of the conventional auxiliary substances and additives, preferably gel formers and membrane stabilisers.

7. Liposomes according to at least one of claims 1 to 6, characterised in that the lipids which form a double layer and chlorhexidine, either as such or dissolved in a small quantity of a water-miscible and suitably physiologically well-tolerated solvent, are combined with an aqueous solution of surfactants and the formation of liposomes in the resulting heterogeneous mixture is initiated by supplying mechanical energy, in particular by shaking, stirring or filtration.

8. The use of liposomes according to one of claims 1 to 7 for the preparation of a medicament for the prophylaxis and/or treatment of bacterial plaque formation, for the chemical treatment of wounds of the gingival pockets, for the treatment of periodically occurring aphthous stomatitis, for the periapical decontamination from bacteria of root canals during endodontal therapy, for the treatment of Candida mycoses of the oral mucosa and periglottis, for the treatment of gingivitis and of gingival hyperplasia and for the disinfection from bacteria of epidermal, mesodermal and mucosal tissue.

## Revendications

1. Liposomes dans lesquels est encapsulé du diacétate de chlorhexidine ou du digluconate de chlorhexidine, comprenant des lipides formant des doubles couches et des agents tensioactifs, caractérisés en ce que les agents tensioactifs sont choisis dans le groupe constitué des acides galliques et de leurs dérivés, ainsi que des sels physiologiquement compatibles de ceux-ci.

2. Liposomes selon la revendication 1, caractérisés en ce que les lipides et/ou les agents tensioactifs sont des composés physiologiquement compatibles, de préférence d'origine naturelle.

3. Liposomes selon la revendication 1 ou la revendication 2, caractérisés en ce que les lipides sont choisis dans le groupe constitué des phospholipides, des sphingolipides et des glycolipides.

4. Liposomes selon l'une au moins des revendications 1 à 3, caractérisés en ce que les agents tensioactifs sont choisis dans le groupe comprenant le cholate de sodium, le désoxycholate de sodium, le glycocholate de sodium, le taurocholate de sodium ou le taurodésoxycholate sodium.

5. Liposomes selon l'une au moins des revendications 1 à 4, caractérisés en ce que le rapport molaire des lipides (L) sur les agents tensioactifs (D) est de 0,5 à 40, de préférence de 2 à 20, et de manière particulièrement préférée de 3,1 à 5,5.

6. Liposomes selon l'une au moins des revendications 1 à 5, caractérisés en ce qu'ils contiennent en plus au moins un des adjuvants et additifs usuels, de préférence des agents gélifiants et des stabilisants de membrane.

7. Liposomes selon l'une au moins des revendications 1 à 6, caractérisés en ce que les lipides formant des doubles couches et la chlorhexidine sont combinés avec une solution aqueuse d'agents tensioactifs, soit tels quels ou soit dissous dans une petite quantité d'un solvant miscible à l'eau et physiologiquement compatible approprié, et en ce que la formation des liposomes est amorcée dans le mélange hétérogène ainsi obtenu par un apport d'énergie mécanique, par exemple par secouage, agitation ou filtration.

8. Utilisation de liposomes selon l'une quelconque des revendications 1 à 7 pour préparer un médicament destiné au traitement préventif et/ou curatif de la formation de plaque bactérienne, au traitement chimique des lésions des poches gingivales, au traitement des inflammations récurrentes de type aphteux de la muqueuse buccale, à la décontamination bactérienne périapicale des canaux radicalaires dans le cadre de thérapies endodontiques, au traitement des mycoses à Candida au niveau des muqueuses de la bouche et de la langue, au traitement de la gingivite et de l'hyperplasie gingivale, et pour la désinfection bactérienne des tissus de l'épiderme, du mésoderme et des muqueuses.
